(19) Europäisches Patentamt
European Patent Office
Office européen des brevets

(11) Veröffentlichungsnummer: **0 374 252 A1**

(12) **EUROPÄISCHE PATENTANMELDUNG veröffentlicht nach Art. 158 Abs. 3 EPÜ**

(21) Anmeldenummer: **88908416.6**

(22) Anmeldetag: **26.05.88**

(86) Internationale Anmeldenummer: **PCT/SU88/00128**

(87) Internationale Veröffentlichungsnummer: **WO 89/11268 (30.11.89 89/28)**

(51) Int. Cl.[5]: **A61K 9/08, A61K 31/22, //(A61K31/22,31:12,31:19,31:25)**

(43) Veröffentlichungstag der Anmeldung: **27.06.90 Patentblatt 90/26**

(84) Benannte Vertragsstaaten: **BE CH DE FR GB IT LI LU NL SE**

(71) Anmelder: **VSESOJUZNY NAUCHNO-ISSLEDOVATELSKY I ISPYTATELNY INSTITUT MEDITSINSKOI TEKHNIKI**
**ul. Kasatkina, 3**
**Moscow, 129301(SU)**

(72) Erfinder: **AKIMOVA, Alla Yakovlevna**
**ul. Petrozavodskaya, 5-3-391**
**Moscow, 125502(SU)**
Erfinder: **DAVYDOV, Anatoly Borisovich**
**ul. Krasny Kazanets, 19-1-283**
**Moscow, 111395(SU)**
Erfinder: **SINEV, Jury Viktorovich**
**Krasnokholmskaya nab., 11/15-1545**
**Moscow, 109172(SU)**
Erfinder: **SOLOMATIN, Alexandr Dmitrievich**
**ul. Nedelina, 40-76**
**Moscow, 121471(SU)**

(74) Vertreter: **von Füner, Alexander, Dr. et al**
**Patentanwälte v. Füner, Ebbinghaus, Finck**
**Mariahilfplatz 2 & 3 Postfach 95 01 60**
**D-8000 München 95(DE)**

(54) **Zusammensetzung zur Blutstillung für die.**

(57) Die vorliegende Erfindung bezieht sich auf ein Gebiet der Medizin.

Die Zusammensetzung zur Blutstillung für die Injektionsanwendung besteht aus folgenden Komponenten in Ma%:

| | |
|---|---|
| ∝-Zyanakrylsäureester oder ein Gemisch der∝-Zyanakrylester | 65,0-75,0 |
| Monoalkylester von Polyäthylenglykol oder von einfachen aliphatischen Alkoholen der allgemeinen Formel: $C_nH_{2n+1}O(C_2H_4)_mH$, worin bedeutet: n = 10-18; m = 7-10 | 0,01-0,02 |
| ein wasserlösliches Keton | 23,29-31,98 |
| Zitronensäure | 1,5-2,5 |
| Tannin | 0,2-0,5 |

EP 0 374 252 A1

SUAA-38728.0

# ZUSAMMENSETZUNG ZUR BLUTSTILLUNG FÜR DIE INJEKTIONSANWENDUNG

## Gebiet der Technik

Die vorliegende Erfindung bezieht sich auf ein Gebiet der Medizin und betrifft insbesondere eine neue Zusammensetzung zur Blutstillung für die Injektionsanwendung.

## Zugrundeliegender Stand der Technik

Es sind Arzneizusammensetzungen zur Stillung der Magendarmblutungen nach der Injektionsmethode in die Submucosa des Magendarmkanals bekannt (Berezov Ju.E. u.a. Stillung der Magendarmblutungen nach der Kombinationsmethode "Khirurgiya" (Chirurgie), 1977, Nr.1, S. 85-89).

Diese Zusammensetzungen sichern jedoch keine standhafte Hämostase wegen einer kurzweiligen Wirkung der Vasokonstriktoren, des Ausbleibens morphologischer Veränderungen in der Blutungszone, was die Möglichkeit einer wiederholten Blutung nach einer kurzen Zeit bedingt.

Es sind auch Zusammensetzung auf Grundlage von öligen Präparten zur Einführung in die Submucosa des Magendarmkanals zwecks der Blutstillung bekannt. Mit diesen Zusammensetzungen führt man während der Endoskopie Lokalinjektionen in der Gegend des blutenden Gefäßes oder Gewebes in vier Abschnitten unter Einführung von 2 bis 4 ml Ölpräparat (ölige Lösungen des Gemisches der Äthylester der Jodphenylundekansäureisomere) in die Submucosa des Magendarmkanals durch. Als Folge der Ausnutzung des Ölpräparats wird ein lange nicht rückbildendes Infiltrat gebildet und es geht die Blutstillung auf Kosten der Gefäßzusammendrückung vor sich (SU, A, 942720).

Die genannten Zusammensetzungen werden dadurch gekennzeichnet, daß bei ihrer Einführung in die Gewebe lange nicht rückbildende Granulome entstehen, die im weiteren die chronische Reizung des Organs aufrechterhalten und zur Entstehung eines neuen pathologischen Prozesses beitragen können. Bei der Einführung in die Submucosa können diese Zusammensetzungen darin wegen ihrer Fließbarkeit und der Porosität der Submucosa umverteilt werden und deswegen hört ihre hämostatische Wirkung auf. Beim Eindringen dieser Präparate in den Gefäßkanal rufen sie die Thrombose auf dem Niveau der Kapillare hervor.

Offenbarung der Erfindung

Der Erfindung liegt die Aufgabe zugrunde, durch die Auswahl entsprechender Komponenten und deren Gewichtsverhältnisse eine Zusammensetzung zur Blutstillung für die Injektionsanwendung zu erhalten, die die Stillung einer massiven Blutung für eine lange Zeit sichert und die Möglichkeit der wiederholten Blutung ausschließt.

Die Aufgabe wurde dadurch gelöst, daß die erfindungsgemäße Zusammensetzung zur Blutstillung für den Injektionsgebrauch erfindungsgemäß aus folgenden Komponenten in Ma% besteht:

| | |
|---|---|
| α-Zyanakrylsäureester oder ein Gemisch der α-Zyanakrylsäureester | 65,0-75,0 |
| Monoalkylester von Polyäthylenglykol und von einfachen aliphatischen Alkoholen der allgemeinen Formel: $C_nH_{2n+1}O(C_2H_4O)_mH$, worin bedeuten: n=10-18, m=7-10 | 0,01-0,02 |
| ein wasserlösliches Keton | 23,29-31,98 |
| Zitronensäure | 1,5-2,5 |
| Tannin | 0,2-0,5 |

Die erfindungsgemäße Zusammensetzung sichert die Möglichkeit deren Injizierung durch die Nadel, der Verbreitung in den Geweben in der Zone von 1 bis 2 cm Radius von der Einführungsstelle, der Polymerisation 1 bis 2 min nach dem Injizieren und der Bildung eines polymeren Blocks, der das blutende Gefäß innerhalb von 15 bis 16 Tagen obliteriert.

Beste Ausführungsvariante der Erfindung

Die Einführung von α-Zyanakrylaten in die Zusammensetzung sichert die Möglichkeit deren Polymerisation beim Kontakt mit den Geweben des Organismus und die Bildung eines polymeren Blocks, der das blutende Gefäß obliteriert. Als α-Zyanakrylate wird ein Gemisch von Äthyl- und Äthoxyäthyl-α-zyanakrylat ausgenutzt, das gestattet, den Termin der Biodestruktion im Bereich von 20 bis 30 Tagen zu regeln.

Infolgedessen obliteriert der sich im Gewebe gebildete polymere Block das blutende Gefäß für eine längere Zeit und schließt die Möglichkeit einer wiederholten Blutung aus.

Das wasserlösliche Keton und Monoalkylester von Polyäthylenglykol und einfachen aliphatischen Alkoholen setzen die Viskosität der Zusammensetzung herab, was gestattet, deren Einführung durch die Nadel zu verwirklichen.

Tannin und Zitronensäure im Bestand der Zusammensetzung sichern die Möglichkeit seiner Injektionseinführung ins Gewebe durch die Nadel.

Die Einführung der Zitronensäure in die Zusammensetzung in einer Menge über 2,5 Ma% führt zur Herabsetzung der Geschwindigkeit der Polymerisation der Zusammensetzung und trägt zu ihrem Ausfluß aus der Injektionszone bei. Die Verminderung des Anteils dieser Komponente führt zur Erhöhung der Geschwindigkeit der Polymerisation der Zusammensetzung, was deren Injizierung durch die Nadel vorbeugt.

Die Einführung der α-Zyanakrylate in die Zusammensetzung in einer Menge unter 65 Ma% führt zur Bildung eines porösen Konglomerats, der die Möglichkeit wiederholter Blutungen nicht ausschließt. Bei der Ausnutzung von α-Zyanakrylaten in einer Menge über 75 Ma% verlängert sich der Termin der Biodestruktion der Zusammensetzung.

Die angebotene Verbindung ist autosteril, übt keine toxische Wirkung auf den Organismus aus. Unter der Wirkung der Organismusmedien geht die Biodestruktion des polymeren Blocks innerhalb von 25 bis 30 Tagen vor sich.

Die erfindungsgemäße Zusammensetzung wurde im Tierversuch und in der Klinik an Menschen untersucht.

Die experimentelle Untersuchung der erfindungsgemäßen Zusammensetzung wurde an 6 bis 12 kg schweren Hunden durchgeführt, Insgesamt wurden 39 Versuche, daraus 25 akute und 14 chronische angestellt. In den akuten Versuchen wurde die Effektivität der Blutstillung auf dem Wege der Einführung der erfindungsgemäßen Zusammensetzung in die Zone der Hämorrhagie durch die Injektionsnadel untersucht. Im chronischen Versuch wurden die Termine der Biodestruktion der Zusammensetzung und die Reaktion der umgebenen Gewebe untersucht.

Alle Untersuchengen wurden unter Narkose durchgeführt. Dazu wurden 15 bis 20 ml 0,%ige Lösung von Natriumthiopental intrapleural eingeführt, nachher wurde der Versuch angestellt.

Im akuten Versucht wurde die Laparotomie mit darauffolgender breiter Gastrotomie in der vorderen Magenwand längs der großen Magenkrümmung durchgeführt. Die Magenblutung wurde durch die Ausschneidung eines Schleimhautabschnittes von verschiedener Tiefe erzeugt. Unter Berücksichtigung der möglichen spontanen Blutstillung am gegebenen Modell einer akuten gastroduodenalen Blutung wurden den Tieren 1000 Einh. Heparin pro 1 kg Körpermasse des Tiers eingeführt. Nachdem eine aktive Blutung hervorgerufen wurde, wurde unmittelbar in die Zone der

Hämorrhagie durch die Injektionsnadel aus 1 bis 2 Punkten von 0,5 bis 1,5 ml erfindungsgemäße Zusammensetzung je nach der Blutungsintensität eingeführt. 30 bis 120 sec nach der Einführung der erfindungsgemäßen Zusammensetzung hörte die Blutung in allen Fällen vollständig auf. Nach der Polymerisation der erfindungsgemäßen Verbindung werden die Gefäße, die sich in der gegebenen Zone befinden, im dichten polymeren Infiltrat zugeklebt. Dadurch wird nicht nur eine zuverlässige Hämostase erreicht, sondern auch die Möglichkeit einer wiederholten Blutung praktisch ausgeschlossen. Im chronischen Versuch wurde den Tieren unter Narkose in die Magensubmucosa durch die endoskopische Nadel, die durch den Arbeitskanal des Fibroendoskops durchgeführt wurde, die Zusammensetzung in einer Menge von 0,5 ml eingeführt. Die Tiere wurden aus dem Versuch durch die intrapleurale Einführung der Natriumthiopentallösung 1, 3, 7 Tage und 2, 3, 4 und 5 Wochen nach der Einführung der erfindungsgemäßen Zusammensetzung hinausgeführt.

Beim Sezieren der Tiere wurden in keinem Fall mikroskopische Merkmale einer Entzündung in der Bauchhöhle und in der Stelle der Einführung der erfindungsgemäßen Zusammensetzung nachgewiesen.

Die mikroskopische Untersuchung der Magengewebe der Tiere einen Tag nach der Einführung der erfindungsgemäßen Zusammensetzung zeigte folgendes: die Unversehrtheit der Schleimhaut wurde nicht verletzt. Es wurden Merkmale der Entzündung der eigenen Plättchen der Schleimhaut und deren basaler Abschnitte verzeichnet. Die Submucosa ist verdickt, mit den Merkmalen von Ödem, Blutergüssen und Leukozyteninfiltration. Die innere Muskelschicht weist die Merkmale eines mäßigen Ödems, der Getrenntheit der Fasern von den vollblutigen Gefäßen, der fokalen intermuskulären Blutergüssen und Leukozyteninfiltraten auf. Die äußere Muskelschicht und die seröse Hülle sind ohne Veränderungen. Am nächsten Tag geht sie Verminderung des Entzündungsgrades vor sich. Zum 7. Tag zeigt die histologische Untersuchung folgendes: in einer geringen Strecke gibt es einen Schleimhautmangel (die Stelle der Einführung der Nadel bei der Injektion), die mit der Infiltrationszone unmittelbar verbunden ist. Die Schleimhaut ist in einer großen Strecke ohne Veränderung. In der Submucosa rings um die erfindungsgemäße Zusammensetzung herum gibt es einen entzündlichen Abgrenzungswall aus polymorphen kernigen Leukozyten mit engen Zwischenschichten von Kollagenfasern im Infiltrat. In den äußeren Abschnitten des Walls sind die Infiltrate vom gleichen Charakter, nur von einem geringeren Grad vorhanden. In den Gefäßen der Submucosa sind die Venen thrombiert. In der Muskelschicht ist die Entzündung nur in der inneren Muskelschicht vorhanden. Im intermuskulären Raum und rings um die Nervengeflechte sind fokale Rundzelleninfiltrate mit den Merkmalen der Lymphostase vorhanden. Die seröse Hülle ist ohne Veränderung.

Nach 5 Wochen laßt sich die erfindungsgemäße Zusammensetzung nict mehr nachweisen, in der Injizierungsstelle werden in der Submucosa fibröse Veränderungen festgestellt. Die seröse und Muskelhülle sind ohne Veränderung.

Die erfindungsgemäße Zusammensetzung kann ausgenutzt werden: - für die Stillung akuter Magendarmblutungen;
- für die Prophylaxe des Rezidivs der Magendarmblutungen;
- für die Prophylaxe der Perforationen und Blutungen während der endoskopischen Entfernung großer gutartiger Geschwülste der hohlen Organe (Magendarmkanal, Bronchialbaum);
- für die endoskopische Schließung der tracheobronchialen Fisteln von 0,4 bis 0,5 cm Gurchmesser.

Die erfindungsgemäße Zusammensetzung würde in der Klinik an 155 Kranken untersucht:
- zwecks der Stillung akuter Magendarmblutungen an 40

Kranken;
- zwecks der Prophylaxe der Rezidive der Magendarmblutungen - an 90 Kranken;
- zwecks der Prophylaxe der Perforationen und Blutungen während der endoskopischen Entfernung großer gatartiger Geschwülste der hohlen Organe - an 20 Kranken;
- zwecks der endoskopischen Schließung der tracheobronchialen Fisteln - an 5 Kranken.

Die erfindungsgemäße Zusammensetzung zur Blutstillung bei den endoskopischen Manipulationen wird durch die endoskopische Nadel mit Hilfe des Fibroendoskops mit der Stirnseitenoptik aufgetragen.

Die erfindungsgemäße Zusammensetzung wurde in der Klinik zwecks der Stillung und Prophylaxe der Magendarmblutungen an 130 Kranken angewendet, daraus sind 25% ältere Kranke und Kranke im hohen Alter. Bei 1 Kranken gelangt es nicht, die Blutung zu stillen. In 7 Fällen (5,4%) erwies sich die Blutstillung als zeitweilig und bei diesen Kranken entstand die wiederholte Blutung. In 5 Fällen von diesen 7 wurde die Blutung durch die wiederholte Anwendung der erfindungsgemäßen Zusammensetzung gestillt.

Der Letalausgang wurde bei 4 Kranken verzeichnet, was 3,1% beträgt.

Zur Zeit beträgt die Mortalität bei akuten gastroduodenalen Blutungen unter Ansnutzung endoskopischer Mittel zur Blutstillung nach den Angaben verschiedener Autoren von 7 bis 20% (durchschnittliche statistische Angaben - 10%) und erreicht bei den Patienten vom höheren Alter 50%. Die Rezidive der gastroduodenalen Blutungen entstehen bei der Ausnutzung existierender endoskopischer Mittel und Methoden in 20 bis 30% der Fälle. Auf diese Weise gestattet die Anwendung der erfindungsgemäßen Zusammensetzung zur Blutstillung die Anzahl der Letalfälle von 10 auf 3,1% herabzusetzen und die von Rezidiven von 20-30 auf 5,4% zu vermindern.

Die erfindungsgemäße Zusammensetzung zur Blutstillung wird durch das Vermischen der bis zum ständigen Gewicht getrockneten Komponenten in der Atmosphäre des trokenen inerten Gases hergestellt und in hermetisch verschlossene Glasfegäße von 5 bis 10 ml Inhalt abgefüllt.

Zum besseren Verständnis der vorliegenden Erfindung werden folgende Beispiele angeführt, die die Varianten der erfindungsgemäßen Zusammensetzung und deren klinischer Prüfung veranschaulichen.

Beispiel 1.

Die erfindungsgemäße Zusammensetzung zur Blutstillung besteht aus folgenden Komponenten, g:

| | |
|---|---|
| Äthyl-α-zyanakrylat | 38,0 |
| Äthoxyäthyl-α-zyanakrylat | 37,0 |
| Dimethylketon | 23,29 |
| Monoalkylester von Polyäthylenglykol und einfachen aliphatischen Alkoholen der Formel $C_nH_{2n+1}O(C_2H_4O)_{10}H$, worin n=10-18 bedeutet | 0,01 |
| Zitronensâure | 1,5 |
| Tannin | 0,2 |

Die Komponenten werden in der Atmosphäre der trockenen Luft vermischt und in hermetisch verschlossene Glasgefäße von 2 ml Inhalt abgefüllt. Die angegebene Zusammensetzung wurde in chronischen Versuchen geprüft. Am offenen Magen wurde ein Schleimhautmangel mit dem Gefäßtrauma von 1 bis 1,5 mm Durchmesser geschaffen, der eine intensive arterielle Blutung hervorrief. Durch die endoskopische Nadel wurden 1,5 ml erfindungsgemäße Zusammensetzung in die Blutungszone eingeführt. Der Radius der Infiltrationszone beträgt 1 cm. 20 sec nach der Einführung der erfindungsgemäßen Zusammensetzung geht deren Polymerisation vor sich. Der sich gebildete polymere Block obliteriert das blutende Gefäß, die Blutung hört auf. Eine wiederholte Blutung wird nicht nachgewiesen. Die Wunde ist schichtweise eingenäht.

Nach 20 Tagen wurden histoligisch die Spuren einer polymeren Plombe nachgewei sen.

Beispiel 2.

Die erfindungsgemäße Zusammensetzung zur Blutstillung besteht aus folgenden Komponenten, g:

| | |
|---|---|
| Äthyl-α-zyanakrylat | 30,0 |
| Äthoxyäthyl-α-zyanakrylat | 35,0 |
| Dimethylketon | 31,98 |
| Monoalkylester von Polyäthylenglykol und einfachen aliphatischen Alkoholen der Formel $C_nH_{2n+1}O(C_2H_4)_8H$, worin n=10-18 bedeutet, | 0,02 |
| Zitronensäure | 2,5 |
| Tannin | 0,5 |

Die genannte Zusammensetzung wurde im Versuch nach der im Beispiel 1 beschriebenen Methodik geprüft.

Der Radius der Infiltrationszone beträgt 2 cm. 60 sec nach der Einführung geht die Polymerisation der Zusammensetzung unter Bildung eines polymeren Blocks vor sich, der das blutende Gefäß zuverlässig obliteriert. Es wurden keine wiederholten Blutungen verzeichnet. Nach 25 Tagen geht die polymere Plombe zurück.

Beispiel 3.

Die erfindungsgemäße Zusammensetzung zur Blutstillung besteht aus folgenden Komponenten, g:

| | |
|---|---|
| Äthyl-α-zyanakrylat | 34,23 |
| Äthoxyäthyl-α-zyanakrylat | 34,23 |
| Dimethylketon | 29,3 |
| Monoalkylester von Polyäthylenglykol und einfachen aliphatischen Alkoholen der Formel $C_nH_{2n+1}O(C_2H_4)_{10}H$, worin n=10-18 bedeutet | 0,01 |
| Zitronensäure | 2,0 |
| Tannin | 0,23 |

Die angegebene Zusammensetzung wurde im Versuch nach der im Beispiel 1 beschriebenen Methodik geprüft.

Der Radius der Infiltrationszone beträgt 1,6 cm. Die Polymerisation der Zusammensetzung und die Bildung des polymeren Blocks, der das blutende Gefäß obliteriert, geht 30 sec nach der Einführung vor sich. Es wird keine wiederholte Blutung verzeichnet. Nach 18 Tagen lassen sich histologisch keine Spuren der polymeren Plombe bestimmen, nach 30 Tagen geht die polymere Plombe zurück.

Beispiel 4.

Es wurden die klinischen Untersuchungen der erfindungsgemäßen Zusammensetzung nach dem Beispiel 3 durchgeführt.

Die Kranke R., 82 Jahre alt, gelangte in die Klinik mit den Merkmalen einer übermäßigen Magendarmblutung. Bei der dringenden Ösophagogastroduodenoskopie wurde festgestellt, daß die Quelle der Blutung der Zerreiß der Schleimhaut im unteren Ösophagusdrittel mit dem Übergang auf die Schleimhaut des Magens von 2,0 cm Länge und 0,5 cm Tiefe ist. Es wurde die Elektrokoagulation der Gewebe durchgeführt und es wurde der Effekt der Blutstillung erhalten. Im weiteren, bei der Beobachtung am nächsten Tag traten die Merkmale einer wiederholten Blutung auf. Bei der endoskopischen Untersuchung wurden die Merkmale der arteriellen Blutung aus der Zerreißstelle festgestellt. Es wurden 2 ml erfindungsgemäße Zusammensetzung nach dem Beispiel 3 in den blutenden Gewebeabschnitt eingeführt. Im weiteren wurden bei der klinischen endoskopischen Beobachtung innerhalb von 20 Tagen keine Merkmale einer wiederholten Blutung verzeichnet.

Beispiel 5.

Der Kranke P., 40 Jahre alt, gelangte in die Klinik mit den Beschwerden über die Schwäche, den Kopfschwindel, den schwarzen Stuhl. Leidet an Ulkuskrankheit des Zwölffingerdarms; früher wurde wegen der akuten gastroduodenalen Blutung die Magenresektion durchgeführt. Beim Kranken wurde der peptische Ulkus der Gastroenteroanastomose festgestellt.

Objektive Angaben: der Zusand des Kranken von der mittleren Schwere, der Puls 92 Schläge pro 1 min, der arterielle Blutdruck 130/80 mm Hg, Hämoglobin 92 g/l, Hämatokrit 30%. Bei der dringenden endoskopischen Untersuchung wurde folgendes festgestellt: Der Magenstumpf enthält eine bedeutende Menge von Blut und Blutgerinnseln. In der Anastomosegegend wurde der Ulkus mit einem aktiv blutenden arteriellen Gefäß bis 1,5 mm Durchmesser nachgewiesen. In die Gegend des blutenden Gefäßes wurden mit Hilfe der endoskopischen Nadel 1,5 ml erfindungsgemäße Zusammensetzung nach dem Beispiel 2 injiziert, nachher hörte die Blutung auf. Bei der endoskopischen Kontrolluntersuchung war der Ulkusboden mit Fibrin bedeckt, die Blutung blieb aus. Im weiteren wurde dem Kranken eine erfolgreiche routinemäßige Behandlung durchgeführt und bei der endoskopischen Untersuchung wurden keine Ulkusmängel in der Anastomosezone verzeichnet. Der Kranke wurde aus der Klinik in einem befriedigenden Zustand entlassen. Beim gegebenen Kranken gestattete die Ausnutzung der erfindungsgemäßen Zusammensetzung nach dem Beispiel 2 die strahlartige arterielle Blutung zu stillen, die sich mit anderen endoskopischen Verfahren nicht stillen ließ (wegen der anatomischen Besonderheiten und einer großen Blutmenge, das aktiv floß und die Quelle der Blutung deckte). Ein dringender operativer Eingriff ist in diesem Fall mit einem hohen Operationsrisiko verbunden, weil dem Kranken die Magenresektion wegen einer akuten gastroduodenalen Blutung schon durchgeführt wurde. Die endoskopische Infiltration mit der erfindungsgemäßen Zusammensetzung gestattete, die dringende Operation mit darauffolgender erfolgreicher routinemäßiger Behandlung zu vermeiden.

Beispiel 6.

Der Kranke K., 55 Jahre alt, gelangte in die Klinik mit den Beschwerden über die Schwäche, den Kopfschwindel, den schwarzen Stuhl. In der Anamnese gibt es keine Ulkuskrankheit.

Objektive Angaben: der Zustand des Kranken von der mittleren Schwere, der Puls 98 Schläge pro 1 min, der Rhythmus ist richtig, arterieller Blutdruck 115/75 Torr, Hämoglobin 72 g/l, Hämatokrit 22%. Das Volumen des zirkulierenden Bluts 3603 ml (bei der Norm von 4380 ml). Bei der dringenden Ösophagogastroduodenoskopie wurde folgendes festgestellt. Der Ösophagus ist ohne Besonderheiten, im Magen ist eine große Menge von Blut und Blutgerinnseln vorhanden. Im oberen Drittel des Magenkörpers nach der großen Magenkrümmung näher zur hinteren Wand wird ein Ulkusmangel von 4x2 $cm^2$ Größe festgestellt. Von dem Boden des Ulkus wird eine strahlartige arterielle Blutung verzeichnet. Mit Hilfe der endoskopischen Nadel wurde in den Grund des blutenden Gefäßes eine Injektion der erfindungsgemäßen Verbindung nach dem Beispiel 1 in einer Menge von 1,5 ml durchgeführt. Unmittelbar nach der Einführung der erfindungsgemäßen Zusammensetzung hörte die Blutung auf. Es wurde eine endoskopische Kontrolluntersuchung vorgenommen, dabei wurde ein Ulkus ohne Merkmale der Blutung nachgewiesen. In der Injektionsstelle ließ sich ein Polymer von 0,3x0,3 $cm^2$ Größe bestimmen. Nach der Präoperationsvorbereitung und der Normalisierung der klinischen Laborangaben wurde die sobtotale Magenresektion nach Hofmeister-Finsterer durchgeführt. Die Postoperationsperiode verlief ohne Komplikationen und der Kranke wurde aus der Klinik im befriedigenden Zustand entlassen.

In diesem Fall gestattete die Anwendung der endoskopischen Infiltration der erfindungsgemäßen Zusammensetzung der chirurgischen dringenden Eingriff zu vermeiden. Es muß hervorgehoben werden, daß der Ausgang des chirurgischen Eingriffs äußerst problematisch sein könnte, weil bei dem Kranken vor seiner Hospitalisierung eine dauernde Blutung (innerhalb von 7 Tagen) vorhanden war, was einen der ungünstigsten Faktoren für die Durchführung des chirurgischen Eingriffs darstellt. Außerdem gestattete die endoskopische Infiltration der erfindungsgemäßen Zusammensetzung die Rezidive der Blutung zu vermeiden und den Kranken zum chirurgischen Planeneingriff vorzubereiten.

Beispiel 7.

Die Kranke S., 50 Jahre alt, gelangte in die Klinik mit der Diagnose: Perforation des Geschlechtsorgans, Peritonits.

Die Kranke wurde dringend operiert. Es wurden das Einnähen des Perforationsulkus des Zwölffingerdarmzwiebels, die Dränage der Bauchhöhle durchgeführt. Die postoperative Periode wurde durch die Unvollständigkeit der Nähte in der Gegend des Einnähens des Ulkus perforans erschwert. Zur enteralen Ernährung mit Hilfe des Endoskops wurde der Nasojejunum-Katheter ausgenutzt. Bei der Kranken trat eine akute gastroduodenale Blutung auf, die sich im mehrmaligen schwarzen flüssigen Stuhl äußerte. Bei der dringenden endoskopischen Untersuchung wurde der Ulkus des Zwölffingerdarmzwiebels von 1,5 bis 2 cm Größe festgestellt, im proximalen Teil läßt sich der Ulkusboden nicht bestimmen (perforative Öffnung). Im distalen Ulkusrand wurde ein pulsierendes thrombiertes Gefäß bis 1,5-2 mm Durchmesser nachgewiesen. Zur Prophylaxe des Rezidivs der Blutung wurde mit Hilfe der endoskopischen Nadel durch den Arbeitskanal des Endoskops in den Gefäsgrund die Injektion der erfindungsgemäßen Zusammensetzung nach dem Beispiel 3 in einer Menge von 1 ml durchgeführt. Bei der weiteren klinischen Beobachtung wurden keine Blutungsrezidive verzeichnet. Der Kranken wurde eine erfolgreiche routinemäßige Behandlung durchgeführt und sie wurde in einem befriedigenden Zustand aus der Klinik entlassen.

Die Anwendung der Methode der Diathermokoagulation (die zur Zeit am effektivsten ist) war im gegebenen Fall unmöglich, weil diese zur Vergrößerung des Öffnungsdurchmessers in der Gegend des Auseinandergehens der Nähte führen würde. Die Ausnutzung der Methode der endoskopischen Infiltration mit der erfindungsgemäßen Zusammensetzung gestattete im gegebenen Fall dem Blutungsrezidiv zuverlässig vorzubeugen und einen dringenden operativen Eingriff zu vermeiden, den die gegebene Kranke nicht vertragen würde.

Beispiel 8.

Der Kranke von 56 Jahre alt. Gelangte in die Klinik mit den Beschwerden über das Vorliegen einer Fistel auf der linken Halsoberfläche. Bei der Beobachtung wurde festgestellt, daß die äußere Fistel mit der Ösophagusöffnung im zervikalen Abschnitt verbunden ist. Die routinemäßige Behandlung der Fistel erwies sich als uneffektiv. Zum Aufhören der Infizierung des Fistelganges seitens des Ösophagus wurde beschlossen, die innere Öffnung der Fistel mit Hilfe der Injektion der erfindungsgemäßen Zusammensetzung nach dem Beispiel 3 in die Gewebe rings um die Fistelöffnung im Ösophagus zu blockieren. Dem Kranken wurde die Fibroösophagoskopie durchgeführt. Im Halsabschnitt des Ösophagus wurde eine Fistelöffnung bis 0,5-0,6 cm Durchmesser festgestellt, durch welche das eitrige Abgesonderte gelangte. Der Fistelgang wurde mit der Chlorhexidinlösung unter darauffolgender Berieselung der Gegend des inneren Fistelganges mit der alkoholischen Chlorhexidinlösung saniert. Danach wurde den Operationskanal eine endoskopische Injektionsnadel durchgeführt. Die Nadel wurde in das Gewebe rings um die Fistel für eine Tiefe von 0,2 bis 0,3 cm eingeführt und in die Gewebe wurde 1,0 ml erfindungsgemäße Zusammensetzung injiziert. Die Öffnung des Fistelganges verengte sich dabei auf 0,2 cm. Die Nadel wurde aus dem Gewebe ausgeführt und von der anderen Seite der Fistelöffnung unter Injizierung noch von 1,5 ml erfindungsgemäße Zusammensetzung wiederum eingeführt. Die Öffnung des Fistelganges ließ sich visuell nicht bestimmen. Bei der Untersuchung des Kranken nach zwei Wochen ließ sich keine Fistelöffnung im Ösophagus bestimmen. Die äußere Fistel wurde geschlossen.

Beispiel 9

Bei der Kranken Ch. im Alter von 45 Jahren wurde bei der Fibrokolonoskopie im S-förmigen Darm ein Polyp von $1{,}5 \times 3{,}0\ cm^2$ Größe auf einem Füßchen festgestellt. Es wurde die Injektion der erfindungsgemäßen Zusammensetzung nach dem Beispiel 2 in einer Menge von 3,0 ml unter den Geschwulstgrund in die Submucosa durchgeführt. Nach der Polymerisation der erfindungsgemäßen Zusammensetzung (nach 2 bis 3 min) wurde die Elektroexzision des Polyps vorgenommen. Die Blutung blieb aus. Bei der Kontrolluntersuchung wurde nach einem Monat in der Stelle der Geschwulstentfernung ein Epithelisationsabschnitt nachgewiesen.

Bei der Anwendung der erfindungsgemäßen Zusammensetzung wird der Möglichkeit der Perforation der Wand eines hohlen Organs sowie der Blutung aus den Gefäßen des Geschwulstbettes nach deren Entfernung vorgebeugt, weil das sich bildende Polymer ein Dielektrikum ist, bei der Einführung die Blutgefäße zusammendrückt und beim Rückgehen innerhalb von 4 bis 5 Wochen der Möglichkeit der Entwicklung der Blutung aus den Gefäßen des Blattes der entfernten Geschwulst in der postoperativen Periode vorgebeugt.

Beispiel 10.

Beim Kranken P. im Alter von 72 Jahren wurde bei der Ösophagogastroskopie eine Geschwulst im mittleren Drittel des Magenkörpers an seiner vorderen Wand bis 5,0 cm Durchmesser festgestellt. Bei der Instrumentenpalpation: die Geschwulst ist von der steinigen Dichte, die Schleimhaut darüber ist beweglich. Unter den Geschwulstgrund wurden 2,0 ml erfindungsgemäße Zusammensetzung nach dem Beispiel 3 eingeführt. Nachher wurde auf den Geschwulstgrund eine endoskopische diametrische Öse umgelegt und es wurde die Ausschneidung der Geschwulst für 1/2 Durchmesser bei deren Grund durchgeführt. Dabei wurde keine Blutung verzeichnet. Bei der wiederholten Untersuchung nach einem Tag wurde die endoskopische Öse auf den Geschwulstgrund aufgelegt, zugezogen und die Geschwulst wurde ausgeschnitten. Bei der Kontrolluntersuchung nach einem Tag ließ sich ein mit Fibrin bedeckter Postkoagulationsmangel bestimmen. Bei der histologischen Untersuchung der Geschwulst wurden die Angaben erhalten, die davon zeugen, daß die Geschwulst ein Fibrom war. Bei der Kontrolluntersuchung wurde nach 2 Monaten in der Stelle der entfernten Geschwulst ein Abschnitt der Hyperämie mit der Konvergenz der Schleimhautfalten dazu nachgewiesen. Bei der Untersuchung des Biopsiestoffes aus der Gegend der entfernten Geschwulst wurden keine Merkmale des Geschwulstwachstums verzeichnet.

## Industrielle Verwendbarkeit

Die erfindungsgemäße Zusammensetzung zur Blutstillung für die Injektionsanwendung findet Verwendung in der Chirurgie und Endoskopie zur Blutstillung und Vorbeugung wiederholter Blutungen.

## PATENTANSPRUCH

Zusammensetzung zur Blutstillung für die Injektionsanwendung dadurch gekennzeichnet, daß diese aus folgenden Komponenten in Ma% besteht:

| | |
|---|---|
| α-Zyanakrylsäureester oder ein Gemisch der α-Zyanakrylsäureester | 65,0 - 75,0 |
| Monoalkylester von Polyäthylenglykol oder von einfachen aliphatischen Alkoholen der allgemeinen Formel: $C_nH_{2n+1}O(C_2H_4)_mH$, worin bedeuten: n=10-18; m=7-10, | 0,01-0,02 |
| ein wasserlösliches Keton | 23,29-31,98 |
| Zitronensäure | 1,5-2,5 |
| Tannin | 0,2-0,5 |

83903416.6

# INTERNATIONAL SEARCH REPORT

00-70

International Application No PCT/SU 88/00128

**I. CLASSIFICATION OF SUBJECT MATTER** (if several classification symbols apply, indicate all) [6]

According to International Patent Classification (IPC) or to both National Classification and IPC

$IPC^4$: A 61 K 9/08, 31/22//(A 61 K 31/22, 31:12, 31:19, 31:25)

**II. FIELDS SEARCHED**

Minimum Documentation Searched [7]

| Classification System | Classification Symbols |
|---|---|
| $IPC^4$: | A 61 K 31/22, 31/275, 31/12, 31/70, 31/19, 31/25, 9/08, A 61 K 45/06 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | FR, A1, 2308360, (ETHOCON, INC.), 19 November 1976, see the claims | 1 |
| A | FR, A1, 2475896, (VSESOJUZNY NAUCHNO-ISSLEDOVATELSKY I ISPYTATELNY INSTITUT MEDITSINSKOI TEKHNIKI) 21 August 1981, see the claims | 1 |
| A | US, A, 4735969, (BASF Aktiengesellschaft), 5 April 1988, see the abstract | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 26 December 1988 (26.12.88) | 24 February 1989 (24.02.89) |
| **International Searching Authority** | **Signature of Authorized Officer** |
| ISA/SU | |

Form PCT/ISA/210 (second sheet) (January 1985)